(19) Europäisches Patentamt
European Patent Office
Office européen des brevets

(11) Veröffentlichungsnummer : **0 139 997**
**B1**

(12) # EUROPÄISCHE PATENTSCHRIFT

(45) Veröffentlichungstag der Patentschrift : **29.07.87**

(51) Int. Cl.⁴ : **C 01 B 25/36**

(21) Anmeldenummer : **84110160.3**

(22) Anmeldetag : **25.08.84**

(54) **Basisches Aluminiumphosphat-Gel, seine Herstellung und Verwendung.**

(30) Priorität : **27.08.83 DE 3330944**

(43) Veröffentlichungstag der Anmeldung : **08.05.85 Patentblatt 85/19**

(45) Bekanntmachung des Hinweises auf die Patenterteilung : **29.07.87 Patentblatt 87/31**

(84) Benannte Vertragsstaaten :
**AT BE CH DE FR GB IT LI LU NL SE**

(56) Entgegenhaltungen :
**DE-A- 2 152 228**
**FR-A- 1 326 148**
**US-A- 4 066 572**

(73) Patentinhaber : **Boehringer Ingelheim International G.m.b.H**
**D-6507 Ingelheim am Rhein (DE)**

(72) Erfinder : **Kniep, Rüdiger, Prof. Dr.**
**Wupperstrasse 26a**
**D-4018 Langenfeld-Richrath (DE)**

**EP 0 139 997 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues basisches Aluminiumphosphat-Gel der bevorzugten mittleren Zusammensetzung

$$Al_6(OH)_{12}(H_2O)_{12}(PO_4)_2,$$

wobei für die Grenzzusammensetzungen das Al : P-Molverhältnis zwischen 2,6 : 1 und 3,3 : 1 liegt, seine Herstellung nach einem neuen Verfahren und seine Verwendung, insbesondere als Antacidum.

Aus der deutschen Patentanmeldung 21 52 228 ist ein therapeutisch wirksames Aluminiumphosphat-Gel bekannt. Im Gegensatz zu dem Gel gemäß der vorliegenden Erfindung weist das bekannte Produkt jedoch ein Al : P-Verhältnis von 1 : 1 auf und ist somit kein basiches Aluminiumphosphat.

Das erfindungsgemäße basische Aluminiumphosphat-Gel hat aufgrund seiner Zusammensetzung und seines Aufbaus besonders günstige therapeutische Eigenschaften. Die Neutralisationswirkung ist aufgrund der basischen Anteile stärker ausgeprägt und es läßt sich im Organismus eine lange anhaltende puffernde Wirkung erzielen.

Außer für die therapeutische Anwendung kommt das erfindungsgemäße Produkt auch für die Anwendung im technischen Bereich in Betracht, wo eine neutralisierende, puffernde oder auch verfestigende (bindende) Wirkung gewünscht wird.

Das erfindungsgemäße basische Aluminiumphosphat-Gel wird hergestellt, indem zunächst die verdünnte wäßrige Lösung eines wasserlöslichen Aluminiumsalzes, vorzugsweise des Salzes einer starken Säure, insbesondere des des Aluminiumchlorids, bis zu einem pH-Wert von 3,3 bis 4, 2, vorzugsweise 3,5-4,0 mit wäßriger Alkalilauge, z. B. Natronlauge, versetzt wird. Es werden dabei ausgedehnte gelöste kationische Aluminiumhydroxid/hydrat-Komplexe unter Erhaltung der oktaedrischen Koordination des Aluminium-Ions vorgebildet. Mit geeigneten Phosphatspezies, insbesondere wasserlöslichen Orthophosphaten starker Basen, z. B. tertiärem Natriumorthophosphat, können die Komplexe dann als elektrisch neutrale Gel-Phasen ausgefällt werden.

Das gesamte Verfahren wird zweckmäßig bei Raumtemperatur durchgeführt.

Beim schonenden Trocknen bleibt der Gelcharakter erhalten. Das Produkt hat eine bevorzugte mittlere Zusammensetzung $Al_6(OH)_{12}(H_2O)_{12}(PO_4)_2$, wobei das Al : P-Verhältnis für die Grenzzusammensetzungen zwischen 2,6 : 1 und 3,3 : 1 liegt.

Das Produkt hat den therapeutisch erwünschten Vorteil, praktisch keine Natrium-Ionen zu enthalten. Es zeigt außerdem eine gute Alterungsbeständigkeit ; nach vierwöchiger Aufbewahrung im geschlossenen Gefäß bei + 40 bis + 50 °C sind keine signifikanten Alterungserscheinungen festzustellen.

Für die Verwendung als Antacidum kann das erfindungsgemäße Produkt in üblicher Weise mit den für diese Zwecke üblichen Hilfs- und Trägerstoffen zu Tabletten, Kapseln, Granulaten, Pulvern, Pasten, Suspensionen verarbeitet werden. Bei der Herstellung wasserhaltiger Formulierungen wie Pasten, Suspensionen braucht das Gel nicht getrocknet zu werden ; es kann nach der Abtrennung von der flüssigen Phase direkt im feuchten Zustand weiterverarbeitet werden.

Tablette

600 Gew.-Teile bas. Aluminiumphosphat-Gel gemäß der Erfindung
100 Gew.-Teile kolloidale Kieselsäure
30 Gew.-Teile Polyvinylpyrrolidon
240 Gew.-Teile Maisstärke
10 Gew.-Teile Magnesiumstearat

Tablette

400 Gew.-Teile bas. Aluminiumphosphat-Gel gemäß der Erfindung
595 Gew.-Teile fettfreies Milchpulver
5 Gew.-Teile Stearinzucker

Die feinteiligen Komponenten werden in üblicher Weise verarbeitet und zu Tabletten von 1 000 mg verpreßt.

Das erfindungsgemäße basische Aluminiumphosphat-Gel kann gewünschtenfalls auch mit anderen Antacida kombiniert werden, z. B. mit Magnesiumcarbonat, -hydroxid, Aluminium/Magnesiumsilikaten, Calciumcarbonat, basischem Wismutnitrat.

Im einzelnen kann das erfindungsgemäße Verfahren nach dem folgenden Beispiel durchgeführt werden :

Beispiel

In eine 1 m wäßrige $AlCl_3 \cdot 6H_20$-Lösung wird innerhalb 30 Minuten bis zum Erreichen eines pH-Wertes von 3,75 ± 0,25 bei 22-25 °C 1 m wäßrige Natronlauge eingeleitet. Man trennt das ausfallende Aluminiumhydroxid ab (es kann wieder in das Chlorid umgewandelt werden). Die klare Lösung, deren Volumen sich bis dahin etwa um den Faktor 2,75 - bezogen auf das ursprüngliche Volumen - vergrößert hat, wird dann innerhalb von 15 Minuten mit einer 0,2 molaren wäßrigen Lösung von $Na_3PO_4 \cdot 12H_2O$ bis zum Erreichen des Neutralpunktes versetzt. Das Volumen hat sich dann gegenüber der ursprünglichen Aluminiumchloridlösung auf etwa das 4,25-fache vergrößert. Bei pH 4,5 ist eine deutliche Viskositätszunahme der Gelsuspension zu beobachten.

Das ausgefällte basische Aluminiumphosphat-Gel wird abfiltriert und chloridfrei gewaschen. Das Produkt wird sodann bei + 40 °C bis zur Gewichtskonstanz getrocknet oder über eine Filterpresse eingeengt. Ausbeute 97 % d. Th. (bezogen auf den Al-Gehalt der vorstrukturierten Lösung).

**Patentansprüche**

1. Basisches Aluminiumphosphat-Gel, dadurch gekennzeichnet, daß die bevorzugte mittlere Zusammensetzung $Al_6(OH)_{12}(H_2O)_{12}(PO_4)_2$ ist und das Al : P-Molverhältnis der Grenzzusammensetzungen zwischen 2,6 : 1 und 3,3 : 1 liegt.

2. Verfahren zur Herstellung eines basischen Aluminiumphosphat-Gels der bevorzugten mittleren Zusammensetzung $Al_6(OH)_{12}(H_2O)_{12}(PO_4)_2$, dadurch gekennzeichnet, daß man ein wasserlösliches Aluminiumsalz in verdünnter wäßriger Lösung langsam mit einer verdünnten wäßrigen Lösung einer starken Base versetzt, bis ein pH-Wert zwischen 3,3 bis 4,2, vorzugsweise 3,5-4,0, erreicht ist und anschließend mit einem tertiären Orthophosphat die Verbindung ausfällt, sie mit Wasser wäscht und schonend trocknet oder abpreßt.

3. Verfahren nach Anspruch 2, dadurch gekennzeichnet, daß man die Reagenzien in etwa einmolaren Lösungen verwendet.

4. Verfahren nach Anspruch 2 oder 3, dadurch gekennzeichnet, daß man von dem Aluminiumsalz einer starken anorganischen Säure ausgeht, vorzugsweise dem Chlorid, als Base Natronlauge und als Phosphat tertiäres Natriumorthophosphat verwendet.

5. Verwendung der Verbindung nach Anspruch 1 als Antacidum.

6. Anticidum, gekennzeichnet durch einen Gehalt an der Verbindung nach Anspruch 1.

**Claims**

1. Basic aluminium phosphate gel characterised in that the preferred average composition is $Al_6(OH)_{12}(H_2O)_{12}(PO_4)_2$ and the molar ratio of Al : P is between 2.6 : 1 and 3.3 : 1.

2. Process for the preparation of a basic aluminium phosphate gel of preferred average composition $Al_6(OH)_{12}(H_2O)_{12}(PO_4)_2$ characterised in that a dilute aqueous solution of a water soluble aluminium salt is slowly mixed with a dilute, aqueous solution of a strong base until a pH of between 3.3 and 4.2, preferably 3.5-4.0, is obtained and the compound is then precipitated out with a tertiary orthophosphate, washed with water and carefully dried or the water extruded.

3. Process according to claim 2, characterised in that approximately one molar solutions of the reagents are used.

4. Process according to claim 2 or 3, characterised in that the starting material is the aluminium salt of a strong inorganic acid, preferably the chloride, sodium hydroxide is used as the base and tertiary sodium orthophosphate is used as the phosphate.

5. Use of the compound according to claim 1 as an antacid.

6. Antacid, characterised in that it contains a compound according to claim 1.

**Revendications**

1. Gel de phosphate d'aluminium basique, caractérisé en ce que la composition moyenne préférée est $Al_6(OH)_{12}(H_2O)_{12}(PO_4)_2$ et en ce que le rapport molaire Al : P des compositions limites se situe entre 2,6 : 1 et 3,3 : 1.

2. Procédé pour la fabrication d'un gel de phosphate d'aluminium basique ayant la composition moyenne préférée $Al_6(OH)_{12}(H_2O)_{12}(PO_4)_2$, caractérisé en ce qu'on additionne lentement un sel d'aluminium soluble dans l'eau en solution aqueuse diluée d'une solution aqueuse diluée d'une base forte, jusqu'à obtention d'une valeur de pH entre 3,3 jusqu'à 4,2, de préférence de 3,5-4,0 et en ce qu'on précipite ensuite le composé au moyen d'un orthophosphate tertiaire, en ce qu'on le lave à l'eau et on le sèche ou soumet à un traitement d'extraction par pression dans des conditions modérées.

3. Procédé selon la revendication 2, caractérisé en ce que l'on utilise les réactifs en des solutions environ une fois molaires.

4. Procédé selon la revendication 2 ou 3, caractérisé en ce qu'on part du sel d'aluminium d'un acide minéral fort, de préférence du chlorure, en ce qu'on utilise comme base la lessive de soude et comme phosphate l'orthophosphate de sodium tertiaire.

5. Utilisation du composé selon la revendication 1 comme agent anti-acide.

6. Agent anti-acide caractérisé par une teneur en le composé selon la revendication 1.